# EUROPEAN PATENT APPLICATION

(11) **EP 1 943 944 A1**
(43) Date of publication of application: **16.07.2008**
(21) Application number: 08100232.1
(22) Date of filing: 09.01.2008
(51) Int. Cl.: A61B 5/0205, A61B 5/04

(54) **Method and arrangement for obtaining diagnostic information of a patient**

(30) Priority: 09.01.2007 US 621213
(71) Applicant: General Electric Company, Schenectady, NY 12345 (US)
(72) Inventor: Laitio, Timo, 20900 Turku (FI); Scheinin, Harry, 20760 Pilspanristi (FI); Huikuri, Heikki, 90100 Oulu (FI); Viertlo-Oja, Hanna, 02660 Espoo (FI); Merilainen, Pekka, 00560 Helsinki (FI)
(74) Representative: Bedford, Grant Richard

(57) **Abstract**

Method and arrangement (2) for obtaining diagnostic information of a patient, the method comprising the steps of carrying out heart rate measurements of the patient (1) at least during two sleep stages before an operation to obtain preoperative heart rate data, carrying out measurements containing information related to the different stages of sleep that are synchronized with the heart rate measurements to obtain preoperative sleep stage data, and calculating a dynamic heart rate variability measures by utilizing non-linear analysis from heart rate data obtained and enabling comparison of the heart rate variability measure between at least two different stages of the sleep state.

## Description

The invention relates generally to a method and an arrangement for obtaining diagnostic information of a patient, for example for predicting perioperative myocardial ischemia.

Patients with myocardial ischemia after non-vascular and non-cardiac vascular surgery have 3 to 9-fold risk of adverse cardiac events, respectively, and cardiac complications account for more than half of the deaths. Especially prolonged ischemia over 10 minutes has been recently shown to be a strong predictor for postoperative death and myocardial infarction. The prevalence of perioperative myocardial ischemia in unselected hip fracture patients has been reported to be over 30 %. The term perioperative refers to time before the operation, under the operation and after the operation. Complications are mainly due to ischemic events, pneumonia and lung embolism. The 3-year mortality rate is over 30 %, and almost half of those who survive are permanently institutionalized.

The autonomic nervous system plays a significant role in the patho-physiology of perioperative ischemia. There is evidence that sympathetic activation has an important role in the onset of adverse cardiac events. Adrenergic activity and plasma catecholamine levels change considerably in the postoperative period, which may predispose to myocardial ischemia by altering relationship between myocardial oxygen demand and supply. Furthermore, increased sympathetic activation during REM sleep has been suggested to be associated with the circadian pattern of ischemia occurring most frequently during early morning hours.

Heart rate variability (HRV) measures from ambulatory electrocardiograph recordings are widely used in the assessment of cardiovascular autonomic regulation. Recent studies suggest that newer dynamic measures of HRV methods can complement the traditional time and frequency domain HRV measures in risk stratification of patients with heart disease. These new dynamic analysis methods describe qualitative rather than quantitative properties of HRV. Various fractal and entropy methods based on fractal mathematics and chaos theory can be used to measure fractal correlation properties, and overall complexity and predictability of the heart rate time series. An article "Fractal dynamics in physiology: Alterations with disease and aging" Goldberger et al, Feb. 19, 2002, can be mentioned as an example describing fractal analysis. Entropy is a concept that describes the irregularity of a signal. Various entropy parameters have been defined and applied to physiological data, such as spectral entropy (Inouye T, Shinosaki K, Sakamoto H, Toi S, Ukai A, lyama A, Katsuda Y, and Hirano M, Quantification of EEG irregularity by use of the entropy of the power spectrum, Electroencephalography and Clinical Neurophysiology, 79 (1991) 204-210), approximate entropy (Pincus SM, Gladstone IM, Ehrenkranz RA, Journal of Clinical Monitoring 1991 Oct; 7(4):335-45), sample entropy (Richman JS, Moorman JR, Physiological time-series analysis using approximate entropy and sample entropy, American Journal of Physiology; Heart and Circulatory Physiology; 2000 Jun;278(6):H2039-49), and multiscale entropy (Costa M, Goldberger AL, Peng CK, Multiscale entropy to distinguish physiologic and synthetic RR time series, Computers in Cardiology, 2002; 29:137-40).

US Published Patent Application No. 2005-0137482-A1 of Laitio et al. describes a method and an arrangement in which fractal correlation properties of heart rate between preoperative day (7-12 AM) and night (2-5 AM) recordings are compared in order to obtain information to predict perioperative myocardial ischemia. It has been shown, however, that as such the fractal correlation properties during REM sleep are very similar to those during wakefulness, whereas the fractal exponent of the variability significantly decreases during non-REM sleep (F. Togo and Y. Yamamoto, Decreased fractal component of human heart rate variability during non-REM sleep, Am J Physiol Heart Circ Phyol 280: H17-H21, 2001), and therefore it is probable that at least in some circumstances the method and the arrangement described in US Published Patent Application No. 2005-0137482-A1 is not an optimal tool to predict myocardial ischemia, i.e. it would be preferable if some improvements could be done.

The American Heart Association has issued guidelines to identify patients at greater risk for postoperative adverse cardiac outcome preoperatively but diagnostic tools with better performance in risk stratification are still needed.

An object of the invention is to meet the guidelines issued by the American Heart Association, i.e. to create a diagnostic tool having better performance in risk stratification.

Various aspects of the invention are based on the idea that the thoughts presented in US Patent Application No. 2005-0137482-A1 are combined with means with which different sleep stages can be distinguished, i.e. the data obtained for example from the awake state and only deep sleep is used, and therefore more accurate prediction of myocardial ischemia and other adverse cardial events (life-threathening arrythmias, myocardial infarction, cardiac death, sudden cardiac death, stroke) is achieved. The invention is characterized in the independent claims 1 and 15. In this invention, the concept of a "sleep stage" refers to stages such as those defined by the Rechtschaffen-Kales sleep scoring rules (Rechtschaffen A and A. Kales, A Manual of Standardized Terminology, techniques and Scoring for Sleep Stages in Human Subjects, Wash. D.C.: U.S. Government Printing Office, 1968. P. 64). These stages include the awake state, the rapid-eye-movement (REM) stage, and the non-REM stages I, II, III an IV.

In the following various aspects and embodiments of the invention will be described in more detail by means of the study carried out and the drawings enclosed in which:
Figure 1 shows a schematic view of the arrangement used in an embodiment of the invention and
Figure 2 shows the results of a sleep measurement over one night.

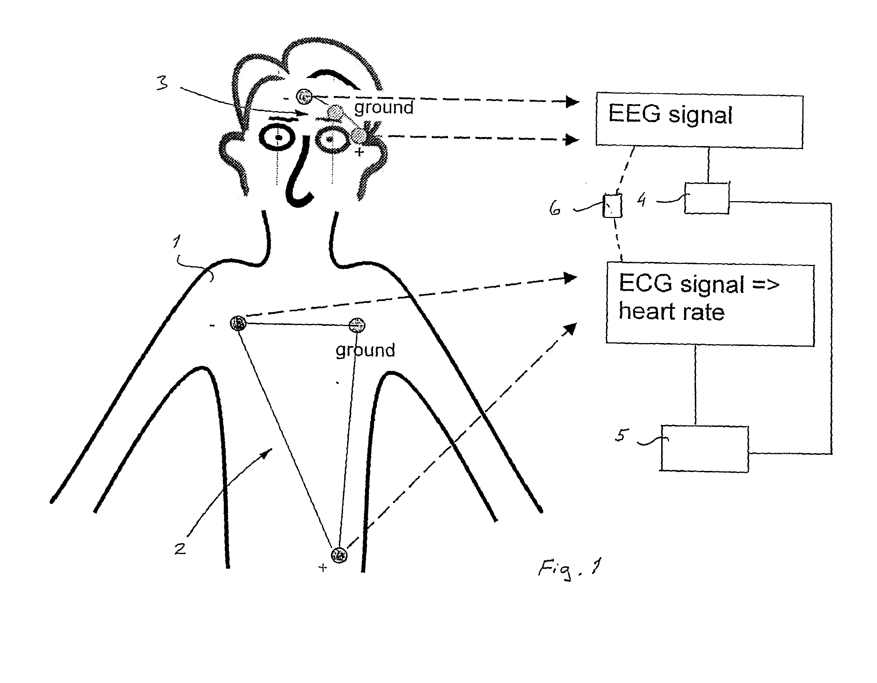

Figure 1 shows a patient 1. Heart rate data can be obtained from the patient for example with two channel Holter recording with an analog device. ECG Holter data can be sampled digitally by using a scanner device and then transferred to a computer for further analysis as described later. A printer can also be connected to the arrangement described. In other words heart rate may be obtained, for example by analyzing the ECG signal. ECG signal can be measured for example between the electrodes (+) and (-) on the chest and abdomen as shown in Figure 1. The arrangement for heart rate measuring is generally shown with reference number 2 in Figure 1. These matters are described for example in the US Patent Application No. 2005-0137482-A1 mentioned above.

According to the basic idea of certain aspects of the invention alongside the heart rate measurement also different stages of the sleep/awake state are distinguished. This can be carried out if also a simultaneous and synchronized EEG measurement is carried out alongside with the heart rate measurement, and the different stages of the sleep/awake state is distinguished by utilizing the EEG data to enable comparison of the heart rate dynamics (e,g. fractal correlation and overall complexity properties of the heart rate time series) between different stages such as, for example, the awake state and deep sleep, or the rapid-eye-movement (REM) sleep and deep sleep. The complexity and predictability of the heart rate time series can be analyzed with various methods determining entropy of the system.

EEG signal is measured between electrodes (+) and (-) On the forehead of the patient as shown in Figure 1. The third electrode in the middle is the ground electrode. The arrangement for EEG measurement is generally shown with reference number 3 in Figure 1. Means 6 enables synchronization of the heart rate data and EEG data.

Determination of the sleep stages would preferably be carried out by an automatic algorithm applying, for example, the concept of spectral entropy (Inouye T, Shinosaki K, Sakamoto H, Toi S, Ukai A, lyama A, Katsuda Y, and Hirano M. Quatification of EEG irregularity by use of the entropy of the power spectrum, Electroencephalography and Clinical Neurophysiology, 79 (1991) 204-210) or the Rechtschaffen-Kales sleep scoring rules.

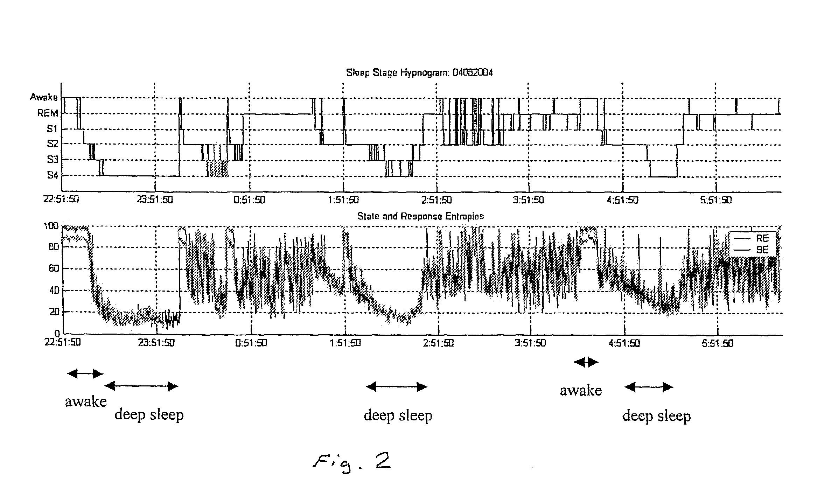

Figure 2 shows the results of a sleep measurement over one night. The horizontal axis corresponds to time.

The plot on top indicates the different stages of sleep according to the Rechtschaffen-Kales sleep scoring system: Awake, rapid-eye movement-sleep (REM), and sleep stages S1 - S4 of which S4 is the deepest stage of sleep. The scoring for this measurement was obtained by performing a simultaneous polysomnographic measurement, which was manually annotated to different sleep stages by a neurophysiologist.

The bottom plot shows State Entropy (SE) and Response Entropy (RE) values as obtained from a measurement using the Entropy^{™} module (GE Healthcare, Helsinki, Finland). The Entropy^{™} module measures the EEG signal from the electrode configuration shown in Figure 1, and calculates the SE and RE values corresponding to the EEG signal. The Entropy^{™} module is schematically shown with reference number 4 in Figure 1. It can be seen that when the patient is in a stage of deep sleep (S3-S4), entropy values are low, whereas when the patient is awake, entropy values are high. Entropy thus provides one possible technique to detect various sleep stages from the measured EEG signal. For example, it is possible to detect the periods during which the patient has been in stable states of wakefulness and deep sleep and select the corresponding epochs of heart rate data for the heart rate variability analysis to represent the two opposite states of the patient.

EEG data classified as told above into different stages of the sleep/awake state is transferred together with heart rate data measured to means configured to carry out dynamic heart rate variability analysis (HRV) between at least two different stages of sleep. The means configured to carry out said dynamic heart rate variability analysis (HRV) is schematically shown with reference number 5 in Figure 1.

According to various aspects of the invention it is preferable that the heart rate measurements are also carried out during awake periods of the patient, and the dynamic heart rate variability measures of the preoperative heart rate data are compared between at least two different stages of the sleep/awake state.

In addition to the EEG data, it is in certain circumstances also advantageous to carry out electromyographic (EMG) and/or electro-oculographic (EOG) measurements and/or electric impedance measurements and to use said data for the classification into different stages of the sleep/awake state. Said EMG and/or EOG and/or impedance measurement can in principle be carried out by using the arrangement shown with reference number 3 in Figure 1.

The heart rate measurement and the heart rate variability analysis (HRV) can be carried out for example in the way as described in in the US Patent Application No. 2005-0137482-A1 mentioned above. The difference between the invention and the method described the US Patent Application No. 2005-0137482-A1 is in that in the invention only the most appropriate parts of the heart rate data obtained is used, and therefore more accurate and reliable tool is obtained when compared to the prior art.

In the following, a possible embodiment of the invention is described in detail. Preoperative two channel continuous Holter recording with a digital device with temporal resolution of at least 500 Hz is applied. Two bipolar leads are used: a modified V5 lead (5^{th} intercostal space at the left mid-clavicular line). The corresponding reference electrodes are in the right and left first intercostal space at the mid-clavicular lines. A horizontal or down-sloping ST-segment depression ≥ 1.0 mm (0.1 mV) or an elevation ≥ 2.0 mm (0.2 mV) at 0.06 sec after the J-point with over 10 minute duration in Holter data are defined as reversible prolonged ischemic changes. All data are also analyzed with short ischemic episodes of at least 1 minute. For each ischemic episode the maximum ST-deviation, its duration, and the area under the ST deviation x time curve (AUC) are determined. The ECG Holter data are sampled digitally and the transferred, together with the EEG data as described above, from the scanner (Oxford Medical Ltd.) to a means, for example a computer, for further analysis of HRV. A careful manual editing of the RR-interval series with inspection of the ECG data by deleting premature beats and noise is performed. All RR-intervals of suspected portions are printed-out on a 2-channel ECG at a paper speed of 25 mm/sec to confirm the sinus origin of all RR-interval data.

Heart rate and standard deviation of all RR-intervals (SDNN) of 24-hour data are used as conventional indices of HRV. Only the most appropriate parts of the heart rate data, i.e. the part of the data obtained by using information based on for example EEG data as described above, is used in HRV analysis. An autoregressive modeling with a model order 20 is used to estimate power spectral densities of RR-interval time series. The power spectra are quantified by measuring the areas in the following frequency bands: very low frequency (VLF) power (0.0033-0.04 Hz), low frequency (LF) power (0.04-0.15 Hz), and high frequency (HF) power. Detrended fluctuation analysis (DFA) is used to quantify fractal-like scaling properties of the time series. Detrended fluctuation analysis is described in www.physionet.org. and also Goldberger AL, Amaral LA, Glass L, Hausdorff JM, Ivanov PC, Mark RG, Mietus JE, Moody GB, Peng CK, Stanley HE (2000). PhysioBank, PysioToolkit, and PhysioNet: components of a new research resource for complex physiologic signals. Circulation 101:E215-220. Shortly, the deviations of each RR intervals from the average RR-interval are integrated over the selected window (1000 beats). Then the window is divided into smaller windows (time scales) and at least squares line fit is applied to the data in each window. This produces a "local" trend, which is subtracted from the overall integrated time series, producing detrended time series. Then a root mean square fluctuation from this integrated and detrended time series is repeatedly calculated using different time scales. Typically, there is a linear relationship between the logarithm of the fluctuation and the logarithm of the size of the time scale. The scaling exponent represents the slope of this line, which relates (log)fluctuation to log(window) size. The present heart rate correlation is defined for short-term fractal-like correlation α1 (window size ≤ 11 beats) of RR-interval data, based on a previous finding of altered short-term heart rate behavior among elderly subjects. An exponent value of 0.5 means that there are no correlations between the RR-intervals as a result of random heart rate dynamics. An exponent value of 1.0 contains both random and highly correlated characteristics in RR-interval time series and has been interpreted to indicate fractal heart dynamics, and has been documented for healthy heart rate dynamics.

Referring to the matters described above it can be said that the fluctuation of the time series in a certain window size is plotted in the function of the window size in a log-log scale. In the case of short-term fractal scaling exponent, the window size is small (e.g. less than 12 beats, the number of beats can be agreed according to the existing need for example). Few window sizes can be used (e.g. 5) and the fluctuation increase as the window size increases in a linear fashion within small window sizes. Finally, the slope of this linear line is calculated. The slope is termed as short-term fractal scaling exponent α1 because in this case the small window sizes were used. Short-term fractal scaling exponent α1 of the DFA method quantifies the fractal-like correlation properties of short-term (for example ≤ 11 beats) RR-interval data.

All preoperative ECG data with ischemic ST-segment changes can be excluded from the HRV analysis. Association of the preoperative HRV measures and the postoperative ischemia can be analyzed further basically in the same way as described in the US Patent Application No. 2005-0137482-A1 mentioned above.

The invention is by no means restricted to the embodiments described above, but the invention can be varied totally freely within the scope of the claims. The invention can be used either in off-line systems or on-line systems, for example in on-line patient monitoring systems. Although the invention is described here referring to perioperative myocardial ischemia, the invention is not restricted to said area but can be used for obtaining any perioperative diagnostic information.

Various aspects and embodiments of the present invention will now be defined in the following numbered clauses:
1. Method for obtaining diagnostic information of a patient, characterized in that the method comprises the steps of:
   carrying out heart rate measurements of the patient at least during two sleep stages before the operation to obtain preoperative heart rate data,
   carrying out measurements containing information related to the different stages of sleep that are synchronized with the heart rate measurements to obtain preoperative sleep stage data, and
   calculating a dynamic heart rate variability measures by utilizing non-linear analysis from heart rate data obtained and enabling comparison of the heart rate variability measure between at least two different stages of the sleep state.
2. The method of clause 1, characterized in that the non-linear analysis is further defined as fractal analysis.
3. The method of any preceding clause, characterized in that the non-linear analysis is further defined as entropy analysis.
4. The method of any preceding clause, characterized in that the measurements containing information related to the different stages of sleep are further defined as encephalographic (EEG) measurements.
5. The method of any preceding clause, characterized in that the heart rate measurements are also carried out during awake periods of the patient, and the dynamic heart rate variability measures are carried out enabling comparison of fractal and/or entropy analyses from the heart rate data obtained from the preoperative heart rate data between at least two different stages of the sleep/awake state.
6. The method of any preceding clause, characterized in that the method further comprises the step of carrying out also electromyographic (EMG) and/or electro-oculographic (EOG) and/or electric impedance data for classifying different stages of the sleep/awake state.
7. The method of any preceding clause, characterized in that obtaining diagnostic information concerns predicting preoperative myocardial ischemia.
8. The method of any preceding clause, characterized in that the fractal correlation properties are short-term fractal correlation properties.
9. The method of any preceding clause, characterized in that the fractal analysis is detrended fluctuation analysis (DFA).
10. The method of clause 9, characterized in that a scaling exponent α1 of the DFA is used as a short-term fractal correlation property.
11. The method of any preceding clause, characterized in that the heart rate measurements and the acquired data are initiated at least a day before the operation.
12. The method of any preceding clause, characterized in that the heart rate measurements are ECG measurements using Holter recording.
13. The method of clause 10, characterized in that the sleep/awake difference of the short-term fractal scaling exponent α1 is determined.
14. The method of any preceding clause, characterized in that comparison of fractal analysis from the heart rate obtained from the preoperative heart rate data between at least two different stages of the sleep state includes the step for finding considerable lowering of the fractal correlation properties before ischemia.
15. The arrangement for obtaining diagnostic information of a patient, characterized in that the arrangement comprises:
   means configured to carry out heart rate measurements of the patient during at least two sleep periods before the operation to obtain preoperative heart rate data,
   means configured to carry out measurements containing information related to the different stages of sleep and configured to enable synchronization of the heart rate data and the sleep stage data in order to obtain preoperative sleep stage data, and
   means configured to calculate a dynamic heart rate variability measure by utilizing non-linear analysis from heart rate data obtained and enabling comparison of the heart rate variability measure between at least two different stages of the sleep state.
16. The arrangement of clause 15, characterized in that the non-linear analysis is further defined as fractal analysis.
17. The arrangement of clause 15 or 16, characterized in that the non-linear analysis is further defined as entropy analysis.
18. The arrangement of clause 15, 16 or 17, characterized in that the measurements containing information related to the different stages of sleep are further defined as encephalographic (EEG) measurements.
19. The arrangement of any of clauses 15 to 18, characterized in that the means configured to carry out heart rate measurements are arranged to carry out heart rate measurements also during awake periods of the patient, and the means configured to generate dynamic heart rate variability measures are arranged to enable comparison of fractal and/or i.e. entropy analysis from the heart rate data obtained from the preoperative heart rate data between at least two different stages of the sleep/awake state.
20. The arrangement of any of clauses 15 to 19, characterized in that the arrangement further comprises further comprises means configured to carry out also electromyographic (EMG) and/or electro-oculographic (EOG) and/or electric impedance data for classifying different stages of the sleep/awake state.
21. The arrangement of any of clauses 15 to 20, characterized in that obtaining diagnostic information concerns predicting preoperative myocardial ischemia.
22. The arrangement of any of clauses 15 to 21, characterized in that the fractal correlation properties are short-term fractal correlation properties.
23. The arrangement of any of clauses 15 to 22, characterized in that the fractal analysis is detrended fluctuation analysis (DFA).
24. The arrangement of any of clauses 15 to 23, characterized in that a scaling exponent α1 of the DFA is used as a short-term fractal correlation property.
25. The arrangement of any of clauses 15 to 24, characterized in that the heart rate measurements and the acquired data are arranged to be initiated at least a day before the operation.
26. The arrangement of any of clauses 15 to 25, characterized in that the heart rate measurements are ECG measurements using Holter recording.
27. The arrangement of any of clauses 15 to 26, characterized in that the sleep/awake difference of the short-term fractal scaling exponent α1 is determined.
28. The arrangement of any of clauses 15 to 27, characterized in that comparison of fractal analysis from the heart rate obtained from the preoperative heart rate data between at least two different stages of the sleep state is arranged to include the step for finding considerable lowering of the fractal correlation properties before ischemia.

## Claims

1. An arrangement (2) for obtaining diagnostic information of a patient (1), **characterized in that** the arrangement comprises:
means configured to carry out heart rate measurements of the patient during at least two sleep periods before the operation to obtain preoperative heart rate data,
means (4) configured to carry out measurements containing information related to the different stages of sleep and configured to enable synchronization of the heart rate data and the sleep stage data in order to obtain preoperative sleep stage data, and
means (5) configured to calculate a dynamic heart rate variability measure by utilizing non-linear analysis from heart rate data obtained and enabling comparison of the heart rate variability measure between at least two different stages of the sleep state.

2. The arrangement (2) of claim 1, **characterized in that** the non-linear analysis is further defined as fractal analysis.

3. The arrangement (2) of any preceding claim, **characterized in that** the non-linear analysis is further defined as entropy analysis.

4. The arrangement (2) of any preceding claim, **characterized in that** the measurements containing information related to the different stages of sleep are further defined as encephalographic (EEG) measurements.

5. The arrangement (2) of any preceding claim, **characterized in that** the means (5) configured to carry out heart rate measurements are arranged to carry out heart rate measurements also during awake periods of the patient, and the means configured to generate dynamic heart rate variability measures are arranged to enable comparison of fractal and/or i.e. entropy analysis from the heart rate data obtained from the preoperative heart rate data between at least two different stages of the sleep/awake state.

6. The arrangement (2) of any preceding claim, **characterized in that** the arrangement further comprises further comprises means configured to carry out also electromyographic (EMG) and/or electro-oculographic (EOG) and/or electric impedance data for classifying different stages of the sleep/awake state.

7. The arrangement (2) of any preceding claim, **characterized in that** obtaining diagnostic information concerns predicting preoperative myocardial ischemia.

8. The arrangement (2) of any preceding claim, **characterized in that** the heart rate measurements and the acquired data are arranged to be initiated at least a day before the operation.

9. The arrangement (2) of any preceding claim, **characterized in that** the heart rate measurements are ECG measurements using Holter recording.

10. A method for obtaining diagnostic information of a patient (1), **characterized in that** the method comprises the steps of:
carrying out heart rate measurements of the patient at least during two sleep stages before the operation to obtain preoperative heart rate data,
carrying out measurements containing information related to the different stages of sleep that are synchronized with the heart rate measurements to obtain preoperative sleep stage data, and
calculating a dynamic heart rate variability measures by utilizing non-linear analysis from heart rate data obtained and enabling comparison of the heart rate variability measure between at least two different stages of the sleep state.
